# FASCICULE DE BREVET EUROPEEN

(11) **EP 1 404 641 B1**
(45) Date de publication et mention de la délivrance du brevet: **06.08.2008**
(21) Numéro de dépôt: 02780917.7
(22) Date de dépôt: 27.06.2002
(51) Int. Cl.: C07C 217/74, C07C 213/10, A61K 31/215

(54) **FORME CRISTALLINE D'UNE PHENYLETHANOLAMINE, SA PREPARATION ET COMPOSITIONS PHARMACEUTIQUES LA CONTENANT**
KRISTALLINE FORM EINES PHENYLETHANOLAMINS, DEREN HERSTELLUNG UND DIESE ENTHALTENDE PHARMAZEUTISCHE ZUSAMMENSETZUNGEN
CRYSTALLINE FORM OF A PHENYLETHANOLAMINE, THE PREPARATION THEREOF AND PHARMACEUTICAL COMPOSITIONS COMPRISING THE SAME

(30) Priorité: 28.06.2001 FR 0108562
(43) Date de publication de la demande: 07.04.2004
(73) Titulaire: Sanofi-Aventis, 75013 Paris (FR)
(72) Inventeur: CARON, Antoine, F-34560 Montbazin (FR); MONNIER, Olivier, F-34560 Villeveyrac (FR); OBERT, Sabrina, F-30250 Sommières (FR); ROCHE, Jérome, F-34730 Prades le Lez (FR); ZIRI, Isabelle, F-34070 Montpellier (FR)
(74) Mandataire: Varady, Peter
(86) Numéro de dépôt international: PCT/FR2002/002235
(87) Numéro de publication internationale: WO 2003/002510

(56) Documents cités:
- EP-A- 0 303 546
- EP-A- 0 489 640
- CECCHI, R. ET AL.: EUR. J. MED. CHEM., vol. 29, 1994, pages 259-267, XP008001333

## Description

La présente invention a pour objet une nouvelle forme cristalline, appelée ci-après forme B, du chlorhydrate de [(7S) 7-[(2R) 2-(3-chlorophényl)-2-hydroxyéthylamino]-5,6,7,8-tétrahydronaphtalen-2-yloxy]-acétate d'éthyle, sa préparation et les compositions pharmaceutiques qui la contiennent.

Ce composé, appelé selon une nomenclature ancienne chlorhydrate de N-[(2S)-7-éthoxycarbonylméthoxy-1,2,3,4-tétrahydronapht-2-yl]-(2R)-(3-chlorophényl)-2-hydroxyéthanamine, et dénommé SR 58611A, ainsi que sa préparation et son activité sur la motricité intestinale ont été décrits dans EP 303546, exemple 12. Par la suite, il a été montré que ce composé possède une activité exceptionnelle sur le système nerveux central, permettant d'envisager son utilisation comme antidépresseur (EP 0489640).

Il a été maintenant trouvé que ce composé existe sous trois formes polymorphes, I, II et III, dénommés ci-après respectivement formes A, B et C, dont l'une, appelée forme B, présente des propriétés physiques avantageuses pour la préparation d'un médicament à l'échelle industrielle.

Le composé SR 58611A est décrit dans l'exemple 12 de EP 0303546 comme un solide vitreux (température de fusion indéterminée), ayant un pouvoir rotatoire de -72,9° (c=0,5%, méthanol), avec 4 valeurs du déplacement par RMN ¹H et 3 pics caractéristiques en IR. Il s'agit ici, comme cela a été trouvé par la suite, d'un mélange des trois polymorphes A, B et C, avec vraisemblablement une majorité de la forme A puisque le pic d'absorption 1203 cm⁻¹ indiqué lui est spécifique (en fait 1206 cm⁻¹ selon une mesure plus précise).

A présent, les trois formes polymorphes A, B, C du composé dénommé SR 58611A peuvent être nettement différenciées :
Points de fusion déterminés par analyse thermique différentielle à balayage (DSC) :
   - forme A (ou forme I) : pF = 158+/-2°C
   - forme B (ou forme II) : pF = 129+/-2°C
   - forme C (ou forme III): pF = 120+/-2°C.
Spectres infrarouges, pics d'absorption caractéristiques :
   - forme A : 2816 cm⁻¹, 2740 cm⁻¹, 1745 cm⁻¹, 1206 cm⁻¹;
   - forme B : 2780 cm⁻¹, 2736 cm⁻¹, 1722 cm⁻¹, 1211 cm⁻¹ ;
   - forme C : 2801 cm⁻¹, 2750 cm⁻¹, 1760 cm⁻¹, 1200 cm⁻¹.
De même, les diagrammes de diffraction des rayons X des poudres de chaque forme sont spécifiques.

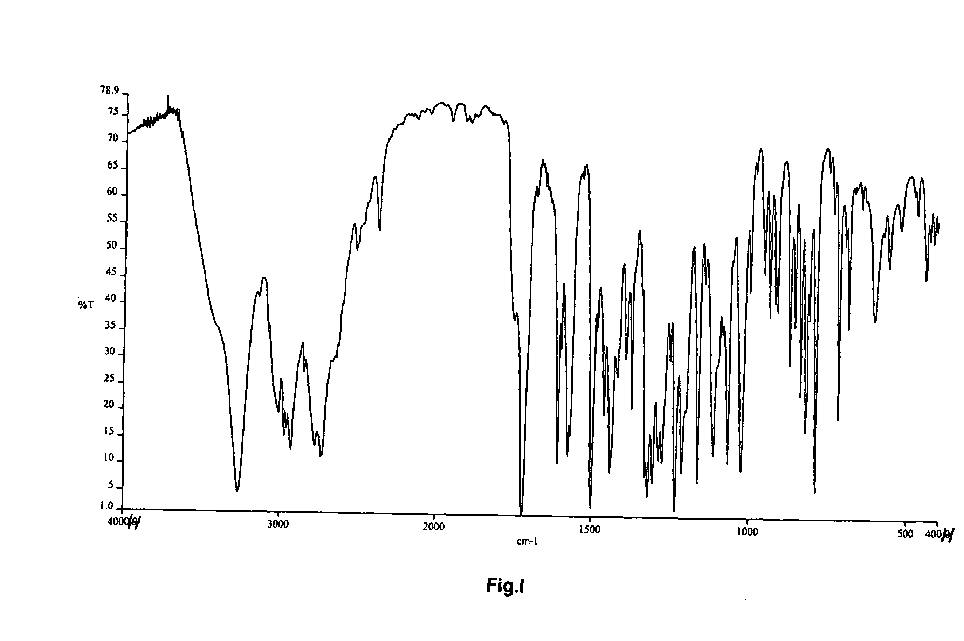

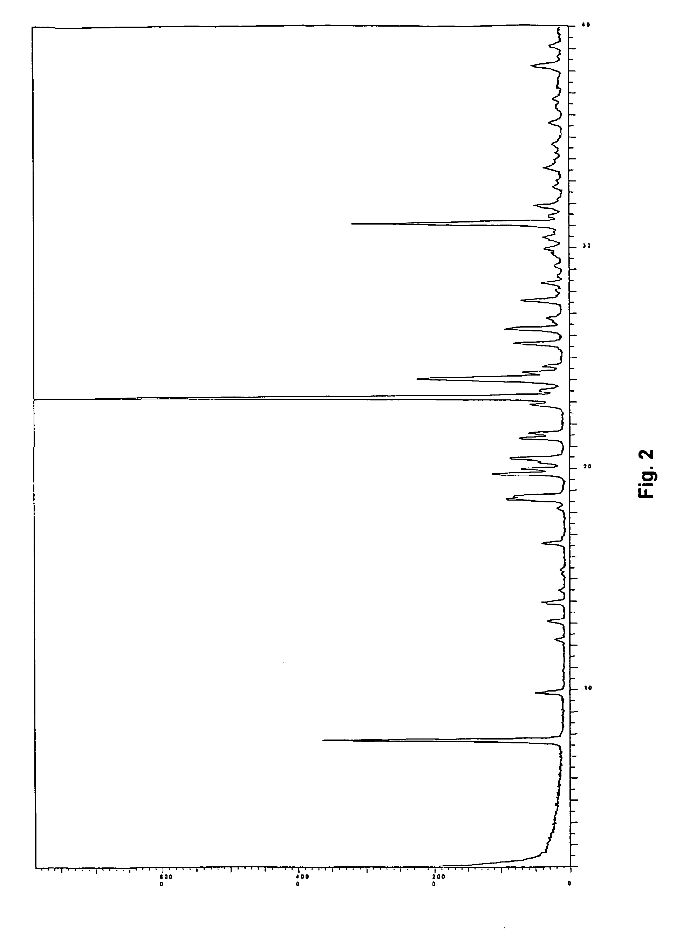

Ainsi, la présente invention a pour objet la forme B du chlorhydrate de [(7S) 7-[(2R) 2-(3-chlorophényl)-2-hydroxyéthylamino]-5,6,7,8-tétrahydronaphtalen-2-yloxy]-acétate d'éthyle qui présente notamment les caractéristiques physiques suivantes :
- pics d'absorption IR caractéristiques (cm⁻¹) : 2780, 2736, 1722, 1211 ;
- point de fusion : 129+/-2°C ;
- raies caractéristiques du diagramme de diffraction des rayons X sur poudre (à 0,1(2θ) près) : 7,69 - 9,83 - 13,95 - 16,58 - 18,70 - 20,40 - 21,57 - 23,40 - 24,15 - 25,64. Le composé est plus particulièrement caractérisé sur les figures 1 et 2 annexées :
- la Figure 1 représente le spectre infrarouge du composé de forme B selon l'invention ; et
- la Figure 2 représente le diagramme de diffraction X sur poudre du composé de forme B (obtenu avec une source Cu Kα 1 dans une configuration θ/θ, monochromateur arrière).

Dans la présente description et les revendications on entend par forme B du SR 58611A ou par composé de l'invention, un produit comprenant au moins 95% en poids et de préférence au moins 99% en poids de forme B à côté des autres polymorphes. Selon un aspect préféré, l'invention a pour objet la forme B essentiellement libre des autres formes polymorphes selon les méthodes d'analyse actuelles.

L'invention comprend aussi un procédé de préparation du composé selon l'invention, caractérisé en ce qu'on ajoute une solution de HCl concentré à une solution de [(7S) 7-[(2R)2-(3-chlorophényl)-2-hydroxyéthylamino]-5,6,7,8-tétrahydronaphtalen-2-yloxy]-acétate d'éthyle dans un solvant choisi parmi le propan-2-ol, le 2-méthylpropan-2-ol et le butan-2-ol à une température allant de 40 à 70°C , on amorce la cristallisation en ensemençant avec une petite quantité de la forme B, puis on refroidit progressivement la solution.

De préférence, la cristallisation est effectuée à une température de 50 à 70°C.

De préférence, ledit solvant est le propan-2-ol.

L'acide chlorhydrique concentré peut être celui du commerce titrant généralement 35 à 38% en poids.

Le composé de l'invention peut aussi être préparé par un procédé de recristallisation, caractérisé en ce qu'on met en suspension le chlorhydrate de [(7S) 7-[(2R)2-(3-chlorophényl)-2-hydroxyéthylamino]-5,6,7,8-tétrahydronaphtalen-2-yloxy]-acétate d'éthyle, sous forme brute ou mélange de formes cristallines, dans le propan-2-ol, le 2-méthylpropan-2-ol ou le butan-2-ol à une température allant de la température ambiante à 70°C et on maintient la suspension en isotherme jusqu'à transformation des cristaux en forme B.

Avantageusement on effectue la recristallisation en amorçant avec une petite quantité de forme B afin de provoquer la nucléation spécifique désirée. Si aucune amorce n'est disponible, il est toutefois possible d'obtenir spontanément la transformation en forme B en maintenant les autres formes cristallines ou les mélanges en suspension notamment dans un alcool tel que mentionné ci-dessus pendant un temps suffisant dépendant de la température, en particulier entre la température ambiante et inférieure à la température d'ébullition de l'alcool.

Selon une autre variante, le produit de l'invention peut être préparé par chauffage d'une solution de chlorhydrate de [(7S) 7-[(2R)2-(3-chlorophényl)-2-hydroxyéthylamino]-5,6,7,8-tétrahydronaphtalen-2-yloxy]-acétate d'éthyle, dans un solvant organique tel qu'un alcool ou une cétone, à une température de 45 à 70°C , concentration de la solution par évaporation ou distillation, puis refroidissement progressif de la solution sous agitation à une température de 10 à 40°C.

En fait, il s'est avéré de manière inattendue que la forme B est la forme la plus stable thermodynamiquement à température ambiante ou plus généralement à une température inférieure ou égale à 70°C, car les autres formes A et C ou leurs mélanges finissent par se transformer en forme B lorsqu'on les maintient en solution ou suspension à une certaine température pendant un temps suffisamment long (voir exemples 3 et 4), quel que soit le solvant. De plus, outre la possibilité d'avoir un produit plus pur et homogène, la forme B peut être obtenue avec une productivité meilleure aussi bien à la cristallisation qu'à la filtration et au séchage, surtout lorsqu'il est préparé dans le propan-2-ol.

Les exemples suivants illustrent l'invention.

### Exemple 1 : Préparation de la forme B du chlorhydrate de SR58611 (SR58611A).

On ajoute 1 équivalent d'acide chlorhydrique concentré à une solution propan-2-olique de SR58611 base (obtenu selon EP0303546) à la concentration de 100 g/l à température ambiante. On chauffe sous agitation le mélange à 70°C pour dissoudre l'ensemble des germes susceptibles de s'être formés et on effectue une rampe de refroidissement linéaire jusqu'à 50°C (+/- 2°C). Sitôt cette température atteinte, on amorce la cristallisation avec 2% de semence SR58611A forme B. On maintient la suspension sous agitation en isotherme à cette température pendant une heure et on refroidit ensuite le milieu par rampe de refroidissement contrôlé jusqu'à 20°C pour permettre une récupération supérieure à 90% du produit sous forme B. Le produit est séparé des eaux mères par filtration, lavé au propan-2-ol et séché à 50°C sous pression réduite pour donner du chlorhydrate SR58611A de forme B, point de fusion 125-130°C. IR conforme.

### Exemple 2 : Recristallisation du chlorhydrate de SR58611.(SR58611A)

Du chlorhydrate de SR58611 mélange de polymorphes (200 g) obtenu selon EP0303546 est solubilisé dans 1,6 litre de propan-2-ol à la température de 80°C. On ajoute 2% (par rapport à la masse de SR58611A introduite) de charbon actif noir CX et on maintient la suspension sous agitation à 80°C pendant 15 minutes. On filtre ensuite le charbon actif et on concentre le filtrat par distillation de 407 volumes de propan-2-ol.

La distillation terminée, sous agitation (Impeller - PN= 120W/m³), on refroidit le milieu homogène à 60°C avec une rampe de refroidissement linéaire de -20°C/h. Sitôt la température de 60°C atteinte, on amorce le milieu avec 2% (par rapport à la masse de SR58611A introduite) de SR58611A forme B en suspension dans de le propan-2-ol.

Après 1 heure d'isotherme à la température d'ensemencement, on refroidit la suspension jusqu'à 20°C avec une rampe de refroidissement linéaire de -10°C/h.

Le produit est séparé par filtration, lavé au propan-2-ol (1 x 400 ml) et séché à 50°C sous pression réduite pour donner du chlorhydrate de SR58611 de forme B (190 g), point de fusion 125-130°C.

### Exemple 3 : Transformation cinétique du SR58611A mélange polymorphe en forme B.

Du chlorhydrate de SR58611 polymorphe (4.46 g) est mis en suspension à température ambiante dans 0,3 litre de propan-2-ol. Après 70 h d'isotherme, la suspension est constituée de chlorhydrate de SR58611A, forme B, >96%. IR et RX conformes.

### Exemple 4 : Transformation cinétique du SR58611A mélange polymorphe en forme B.

Du chlorhydrate de SR58611 polymorphe (95.2 g) est mis en suspension à 70°C dans 0,3 litre de propan-2-ol. Après 12h30 d'isotherme, la suspension est constituée de chlorhydrate de SR58611, forme B, >98%. IR et RX conformes.

### Exemple 5 : Préparation industrielle du SR58611A de forme B.

On charge un réacteur sec et purgé par azote avec 24,7 kg de SR58611A mélange de polymorphes (pF=159°C), 185 I de propan-2-ol et on chauffe jusqu'à 75°C (la température de dissolution totale est 68°C). On introduit ensuite 0,49kg charbon actif CX et 5 I. de propan-2-ol et on porte la température à 80°C pendant 30 minutes. Ensuite on filtre le contenu maintenu à 75-78°C par pression d'azote , on rince la cuve avec 7 I. de solvant et on maintient encore 10 minutes à 78°C, puis on concentre le milieu sous pression atmosphérique jusqu'à un volume résiduel de 99 I. en env. 1h. On refroidit le milieu jusqu'à 60°C (vitesse 0 ?4°C/min.) et on introduit immédiatement 0,5kg d'amorce (forme B) avec 2,5 I. de propan-2-ol et on maintient à 60°C pendant une heure . On refroidit à 20 °C (vitesse 0,2°C/min), puis on filtre, lave avec 25 I. de propan-2-ol et sèche sous vide à 40°C puis 70°C jusqu'à une perte de poids < 0,1 %. On obtient 23,04kg du produit attendu (Rt ; 93,3%). I R et RX conformes.

### Exemple 6 : Comparaison de séchage

Le séchage, en filtre sécheur agité, d'un gâteau de filtration humide de SR58611A de forme A conduit en moins de deux heures à un changement de forme cristalline et à l'obtention d'un gâteau constitué de 90% de forme C. Pour revenir à la forme A, il faut alors continuer le séchage à une température supérieure à 60°C sous agitation pendant environ 48h.

La forme B présente l'intérêt de ne pas changer de forme cristalline au séchage. Par ailleurs, la forme des cristaux obtenus (plaquettes hexagonales épaisses) permet de réduire l'humidité résiduelle du gâteau de filtration à des teneurs inférieures à 20%, et facilite de fait, le séchage du produit en vue de son utilisation comme principe actif de médicament.

### Exemple 7 : Composition pharmaceutique.

Le produit selon l'invention peut être administré pour le traitement de la dépression à une dose allant de 100 à 800 mg/jour (calculé en base), notamment 300 à 600 mg/jour par voie orale, selon la sévérité de la maladie et le poids du patient.

Des formulations typiques sont les comprimés ou les gélules ou dragées. Exemples de gélules dosées à 100 et 200mg de principe actif (en base, correspondant respectivement à 109,0 et 218,0mg de chlorhydrate) :

| | | |
|---|---|---|
| SR58611A forme B | 100 | 200 |
| Lactose monohydrate | 330,5(QS) | 217,3 |
| Hydroxypropylméthylcellulose | 4,2 | 8,4 |
| Stéarate de magnésium | 6,3 | 6,3 |
| Total (mg) | 450 | 450 |

## Revendications

1. Forme B du chlorhydrate de [(7S) 7-[(2R) 2-(3-chlorophényl)-2-hydroxyéthylamino]-5,6,7,8-tétrahydronaphtalen-2-yloxy]-acétate d'éthyle dont le spectre infrarouge présente les pics d'absorption caractéristiques suivants : 2780, 2736, 1722, 1211 cm⁻¹.

2. Forme B du chlorhydrate de [(7S) 7-[(2R) 2-(3-chlorophényl)-2-hydroxyéthylamino]-5,6,7,8-tétrahydronaphtalen-2-yloxy]-acétate d'éthyle, ayant un point de fusion de 129+/-2°C déterminé par analyse thermique différentielle à balayage.

3. Forme B du chlorhydrate de [(7S) 7-[(2R) 2-(3-chlorophényl)-2-hydroxyéthylamino]-5,6,7,8-tétrahydronaphtalen-2-yloxy]-acétate d'éthyle dont le diagramme de diffraction des rayons X de poudre présente les raies caractéristiques suivantes : 7,69 - 9,83 - 13,95 - 16,58 - 18,70 - 20,40 - 21,57 - 23,40 - 24,15 - 25,64.

4. Procédé de préparation du composé selon les revendications 1 à 3, **caractérisé en ce qu'**on ajoute une solution de HCl concentré à une solution de [(7S) 7-[(2R) 2-(3-chlorophényl)-2-hydroxyéthylamino]-5,6,7,8-tétrahydronaphtalen-2-yloxy]-acétate d'éthyle dans un solvant choisi parmi le propan-2-ol, le 2-méthylpropan-2-ol et le butan-2-ol à une température allant de 40 à 70°C , on amorce la cristallisation en ensemençant avec une petite quantité de la forme B., puis on refroidit progressivement la solution.

5. Procédé selon la revendication 4, **caractérisé en ce qu'**on effectue la cristallisation à une température de 50 à 70°C.

6. Procédé selon la revendication 4 ou 5, **caractérisé en ce que** le solvant est le propan-2-ol.

7. Procédé de préparation du composé selon les revendications 1 à 3, **caractérisé en ce qu'**on met en suspension le chlorhydrate de [(7S) 7-[(2R) 2-(3-chlorophényl)-2-hydroxyéthylamino]-5,6,7,8-tétrahydronaphtalen-2-yloxy]-acétate d'éthyle dans le propan-2-ol, le 2-méthylpropan-2-ol et le butan-2-ol à une température allant de la température ambiante à 70°C et on maintient la suspension en isotherme jusqu'à transformation des cristaux en forme B.

8. Procédé de préparation du composé selon les revendications 1 à 3, **caractérisé en ce qu'**on chauffe une solution de chlorhydrate de [(7S) 7-[(2R) 2-(3-chlorophényl)-2-hydroxyéthylamino]-5,6,7,8-tétrahydronaphtalen-2-yloxy]-acétate d'éthyle dans un solvant organique choisi parmi un alcool ou une cétone, à une température de 45 à 70°C, on concentre la solution par évaporation ou distillation, puis on refroidit progressivement la solution sous agitation à une température de 10 à 40°C.

9. Procédé selon la revendication 8, **caractérisé en ce que** le solvant est le propan-2 -ol.

10. Composition pharmaceutique comprenant comme principe actif le composé selon les revendications 1 à 3, ainsi qu'un ou plusieurs excipients pharmaceutiquement acceptables.

11. Médicament **caractérisé en ce qu'**il comprend le composé selon les revendications 1 à 3.

## Claims

1. B form of ethyl [(7S)-7-[(2R)-2-(3-chlorophenyl)-2-hydroxyethylamino]-5,6,7,8-tetrahydronaphth-2-yloxy]acetate hydrochloride, the infrared spectrum of which exhibits the following characteristic absorption peaks: 2780, 2736, 1722, 1211 cm⁻¹.

2. B form of ethyl [(7S)-7-[(2R)-2-(3-chlorophenyl)-2-hydroxyethylamino]-5,6,7,8-tetrahydronaphth-2-yloxy]acetate hydrochloride having a melting point of 129+/-2°C, determined by differential scanning calorimetry.

3. B form of ethyl [(7S)-7-[(2R)-2-(3-chlorophenyl)-2-hydroxyethylamino]-5,6,7,8-tetrahydronaphth-2-yloxy]acetate hydrochloride, the powder X-ray diffraction diagram of which exhibits the following characteristic lines: 7.69, 9.83, 13.95, 16.58, 18.70, 20.40, 21.57, 23.40, 24.15 and 25.64.

4. Process for the preparation of the compound according to Claims 1 to 3, **characterized in that** a solution of concentrated HCl is added to a solution of ethyl [(7S)-7-[(2R)-2-(3-chlorophenyl)-2-hydroxyethylamino]-5,6,7,8-tetrahydronaphth-2-yloxy]acetate in a solvent chosen from propan-2-ol, 2-methylpropan-2-ol and butan-2-ol at a temperature ranging from 40 to 70°C, crystallization is initiated by seeding with a small amount of the B form and then the solution is gradually cooled.

5. Process according to Claim 4, **characterized in that** the crystallization is carried out at a temperature of 50 to 70°C.

6. Process according to Claim 4 or 5, **characterized in that** the solvent is propan-2-ol.

7. Process for the preparation of the compound according to Claims 1 to 3, **characterized in that** ethyl [(7S)-7-[(2R)-2-(3-chlorophenyl)-2-hydroxyethylamino]-5,6,7,8-tetrahydronaphth-2-yloxy]acetate hydrochloride is suspended in propan-2-ol, 2-methylpropan-2-ol -or. butan-2-ol at a temperature ranging from ambient temperature to 70°C and the suspension is maintained under isothermal conditions until the crystals have been converted to the B form.

8. Process for the preparation of the compound according to Claims 1 to 3, **characterized in that** a solution of ethyl [(7S)-7-[(2R)-2-(3-chlorophenyl)-2-hydroxyethylamino]-5,6,7,8-tetrahydronaphth-2-yloxy]acetate hydrochloride in an organic solvent chosen from an alcohol or a ketone is heated to a temperature of 45 to 70°C, the solution is concentrated by evaporation or distillation and then the solution is gradually cooled with stirring to a temperature of 10 to 40°C.

9. Process according to Claim 8, **characterized in that** the solvent is propan-2-ol.

10. Pharmaceutical composition, comprising the compound according to Claims 1 to 3 as active principle and one or more pharmaceutically acceptable excipients.

11. Medicament, **characterized in that** it comprises the compound according to Claims 1 to 3.

## Patentansprüche

1. B-Form des Hydrochlorids von [(7S)-7-[(2R)-2-(3-Chlorphenyl)-2-hydroxyethylamino]-5,6,7,8-tetrahydronaphthalin-2-yloxy]-ethylacetat, deren Infrarotspektrum folgende charakteristische Absorptionsmaxima aufweist: 2780, 2736, 1722, 1211 cm⁻¹.

2. B-Form des Hydrochlorids von [(7S)-7-[(2R)-2-(3-Chlorphenyl)-2-hydroxyethylamino]-5,6,7,8-tetrahydronaphthalin-2-yloxy]-ethylacetat, deren mittels dynamischer Differenzkalorimetrie bestimmter Schmelzpunkt 129 ± 2°C beträgt.

3. B-Form des Hydrochlorids von [(7S)-7-[(2R)-2-(3-Chlorphenyl)-2-hydroxyethylamino]-5,6,7,8-tetrahydronaphthalin-2-yloxy]-ethylacetat, deren Röntgenpulverbeugungsdiagramm folgende charakteristische Absorptionslinien aufweist: 7,69 - 9, 83 - 13,95 - 16,58 - 18,70 - 20,40 - 21,57 - 23,40 - 24,15 - 25,64.

4. Verfahren zur Herstellung der Verbindung nach den Ansprüchen 1 bis 3, **dadurch gekennzeichnet, dass** man eine konzentrierte HCl-Lösung zu einer Lösung von [(7S)-7-[(2R)-2-(3-Chlorphenyl)-2-hydroxyethylamino]-5,6,7,8-tetrahydronaphthalin-2-yloxy]-ethylacetat in einem Lösungsmittel, ausgewählt aus Propan-2-ol, 2-Methylpropan-2-ol und Butan-2-ol, bei einer Temperatur von 40 bis 70°C hinzu gibt, die Kristallisation durch Beimpfen mit einer kleinen Menge der B-Form einleitet und anschließend die Lösung nach und nach abkühlt.

5. Verfahren nach Anspruch 4, **dadurch gekennzeichnet, dass** man die Kristallisation bei einer Temperatur von 50 bis 70°C durchführt.

6. Verfahren nach Anspruch 4 oder 5, **dadurch gekennzeichnet, dass** das Lösungsmittel Propan-2-ol ist.

7. Verfahren zur Herstellung der Verbindung nach den Ansprüchen 1 bis 3, **dadurch gekennzeichnet, dass** man das Hydrochlorid von [(7S)-7-[(2R)-2-(3-Chlorphenyl)-2-hydroxyethylamino]-5,6,7,8-tetrahydronaphthalin-2-yloxy]-ethylacetat in Propan-2-ol, 2-Methylpropan-2-ol und Butan-2-ol bei einer Temperatur von Raumtemperatur bis 70°C suspendiert und die Suspension bis zur Umwandlung der Kristalle in die B-Form isotherm hält.

8. Verfahren zur Herstellung der Verbindung nach den Ansprüchen 1 bis 3, **dadurch gekennzeichnet, dass** man eine Lösung des Hydrochlorids von [(7S)-7-[(2R)-2-(3-Chlorphenyl)-2-hydroxyethylamino]-5,6,7,8-tetrahydronaphthalin-2-yloxy]-ethylacetat in einem organischen Lösungsmittel, ausgewählt aus einem Alkohol oder einem Keton, auf eine Temperatur von 45 bis 70°C erwärmt, die Lösung mittels Verdampfen oder Destillation einengt und anschließend die Lösung unter Rühren allmählich auf eine Temperatur von 10 bis 40°C abkühlt.

9. Verfahren nach Anspruch 8, **dadurch gekennzeichnet, dass** das Lösungsmittel Propan-2-ol ist.

10. Pharmazeutische Zusammensetzung, die als Wirkstoff die Verbindung nach den Ansprüchen 1 bis 3 sowie einen oder mehrere pharmazeutisch unbedenkliche Exzipienten umfasst.

11. Arzneimittel, **dadurch gekennzeichnet, dass** es die Verbindung nach den Ansprüchen 1 bis 3 umfasst.
